# EUROPÄISCHE PATENTANMELDUNG

(11) **EP 2 557 774 A1**
(43) Veröffentlichungstag der Anmeldung: **13.02.2013**
(21) Anmeldenummer: 11177102.8
(22) Anmeldetag: 10.08.2011
(51) Int. Cl.: H04N 5/335

(54) **Bildgebende Vorrichtung, endoskopisches Instrument, Verfahren zum Betreiben einer bildgebenden Vorrichtung und Verwendung einer bildgebenden Vorrichtung**

(71) Anmelder: Karl Storz GmbH & Co. KG, 78532 Tuttlingen (DE)
(72) Erfinder: Baum, Eckhart, 78589 Dürbheim (DE); Schwarz, Peter, 78532 Tuttlingen-Nendingen (DE)
(74) Vertreter: Witte, Weller & Partner

(57) **Zusammenfassung**

Bildgebende Vorrichtung (16) für ein endoskopisches Instrument (10), die Vorrichtung (16) mit einem Bildsensor (24), der eine Ausleseeinrichtung (26) zum Auslesen einer bildgebenden Fläche (32) des Bildsensors (24) aufweist, wobei die Ausleseeinrichtung (26) dafür ausgebildet ist, ein erstes digitales Datensignal (34) mit Nutzdaten (36) zu erzeugen, der einen ersten Anteil (P1) einer ersten Nutzdatendauer (T1) bezogen auf eine Auslesedauer (TR) aufweist, und mit einer Verarbeitungseinrichtung (38), die dafür ausgebildet ist, auf der Basis des ersten Datensignals (34) ein zweites digitales Datensignal (40) zu erzeugen, bei dem zumindest ein Teil der Nutzdaten (36) zeitlich gestreckt ist, so dass ein zweiter Anteil (P2) einer zweiten Nutzdatendauer (T2) an der Auslesedauer (TR) größer ist als der erste Anteil (P1). Ferner werden ein endoskopisches Instrument (10) mit einer bildgebenden Vorrichtung (16), ein Verfahren zum Betreiben einer bildgebenden Vorrichtung (16) und die Verwendung einer bildgebenden Vorrichtung (16) offenbart.

## Beschreibung

Die Erfindung betrifft eine bildgebende Vorrichtung für ein endoskopisches Instrument, wobei die Vorrichtung einen Bildsensor aufweist, der eine Ausleseeinrichtung zum Auslesen einer bildgebenden Fläche des Bildsensors aufweist, und wobei die Ausleseeinrichtung dafür ausgebildet ist, ein erstes digitales Datensignal mit Nutzdaten zu erzeugen.

Die Erfindung betrifft des Weiteren ein endoskopisches Instrument mit einem Schaft, der ein distales Ende, an dem mindestens eine bildgebende Vorrichtung angeordnet ist, und ein proximales Ende zum Anschluss an eine Versorgungseinheit aufweist, und mit einem Datenübertragungselement, das zwischen der mindestens einen bildgebenden Vorrichtung und dem proximalen Ende angeordnet ist. Die Erfindung betrifft außerdem ein Verfahren zum Betreiben einer bildgebenden Vorrichtung und die Verwendung einer bildgebenden Vorrichtung.

Derartige bildgebende Vorrichtungen und endoskopische Instrumente sind bekannt und werden bspw. bei einem Video-Gastroskop mit Gastro Pack® von der Firma Karl Storz GmbH & Co. KG vertrieben.

Grundsätzlich ist es bekannt und seit einiger Zeit üblich, endoskopische optische bzw. optoelektronische Sensoren aus CCD (Charge-Coupled-Device)- oder CMOS (Complementary Metal Oxide Semiconductor)-Modulen aufzubauen. Die Anforderungen an geringe Dimensionen des endoskopischen Instruments erlauben es in der Praxis üblicherweise nicht, Bilddatenverarbeitungs- und Steuerungseinheiten dieser Sensoren an dem distalen Ende des endoskopischen Instruments vorzusehen. Daher sind Datenübertragungselemente angeordnet, die die Bilddaten von den optischen Sensoren zu einer Versorgungseinheit übertragen und umgekehrt Steuersignale bzw. Ansteuertakte von der Versorgungseinheit an den optischen Sensor bzw. Bildsensor übermitteln.

Im Telekommunikationsbereich, insbesondere im Bereich der Mobiltelefone, wurden CMOS-Sensoren entwickelt, deren Abmessungen zwar so gering sind, dass sie in eine Endoskopspitze integriert werden könnten, die Bildsensoren aber dennoch eine hohe Auflösung, insbesondere eine HD-Auflösung, besitzen. Etliche dieser Bildsensoren besitzen bereits einen oder mehrere A/D-Wandler, so dass die Rohbilddaten bzw. Rohvideodaten (Nutzdaten) in digitaler Form parallel oder seriell ausgegeben werden können.

Dazu werden die einzelnen Pixel der bildgebenden Fläche bzw. aktiven Fläche des Bildsensors nacheinander abgetastet, in ein digitales Signal umgesetzt und über eine digitale Schnittstelle zur weiteren Verarbeitung ausgegeben. Die Ausgabe der Nutzdaten erfolgt dabei synchron zum Pixelauslesetakt. Zur Formatierung der Daten, wie z.B. der Aufsplittung von 10-Bit-Pixeldaten in einzelne Byte-Pakete ist auf dem Sensor ein kleiner Speicher, üblicherweise ein FIFO (First-In-First-Out) integriert.

Bei der Verwendung der zuvor genannten Bildsensoren in einem Endoskop stößt man bei der praktischen Realisierung jedoch auf Probleme. Die Probleme sind hauptsächlich darin begründet, dass die Bildsensoren innerhalb eines Mobiltelefons über eine sehr kurze Leitung, üblicherweise wenige Zentimeter, mit der entsprechenden Verarbeitungseinheit verbunden sind. Die Datenübertragung ist daher auch bei hohen Datenübertragungsraten, bspw. ein Gbit/s und mehr, völlig unproblematisch.

Im Vergleich hierzu beträgt die Länge der Leitung, bzw. allgemein des Datenübertragungselements, regelmäßig mehr als 50 cm, oft mehr als 100 cm und kann auch mehr als 150 cm betragen. Dies bedeutet konkret, dass die Dämpfung eines Datenübertragungselements innerhalb eines endoskopischen Instruments wesentlich höher ist als die der Leitung in einem Mobiltelefon - einen gleichbleibenden Leitungsdurchmesser vorausgesetzt.

Um Signale dennoch zuverlässig auch über ein längeres Datenübertragungselement übertragen zu können, muss der Durchmesser der Leitung bzw. des Datenübertragungselements vergrößert werden. Gerade in der Endoskopie ist der Durchmesser eines Datenübertragungselements aber stark limitiert, da bei der Anwendung am Menschen bestimmte Maximaldurchmesser des endoskopischen Instruments nicht überschritten werden dürfen. Dies alles führt im Ergebnis dazu, dass die heutige Verwendung von kostengünstigen Bildsensoren nur mit Einschränkungen, sei es hinsichtlich der Qualität oder der Funktionalität, oder aber überhaupt nicht möglich ist.

Naheliegend zeigt sich folgende Lösungsmöglichkeit zur Behebung dieses Problems. Es können spezielle Bildsensoren entwickelt werden, die bspw. nur eine 70%-HD-Auflösung bereitstellen. Die Entwicklung und Fertigung solcher speziellen Bildsensoren ist jedoch sehr teuer. Zudem fehlt es bei derart speziellen Sensoren an breiter praktischer Erfahrung und gewachsenem Vertrauen in eine dauerhafte Zuverlässigkeit. Schließlich wird von den Anwendern auch "Full HD" eingefordert, um im Operationssaal eine optimale Bildqualität zu haben.

Es ist daher eine Aufgabe der vorliegenden Erfindung, eine Möglichkeit aufzuzeigen, die aus dem Telekommunikationsbereich bekannte Bildsensoren und digitalen Signalschnittstellen auch auf dem Gebiet der Endoskopie unter Beachtung der Dimensionen der Instrumente und Kabeldurchmesser nutzen zu können.

Diese Aufgabe wird nach einem Aspekt der Erfindung gelöst durch eine bildgebende Vorrichtung für ein endoskopisches Instrument, wobei die Vorrichtung einen Bildsensor aufweist, der eine Ausleseeinrichtung zum Auslesen einer bildgebenden Fläche des Bildsensors aufweist, wobei die Ausleseeinrichtung dafür ausgebildet ist, ein erstes digitales Datensignal mit Nutzdaten zu erzeugen, der einen ersten Anteil einer ersten Nutzdatendauer bezogen auf eine Auslesedauer aufweist, und wobei die Vorrichtung eine Verarbeitungseinrichtung aufweist, die dafür ausgebildet ist, auf der Basis des ersten Datensignals ein zweites digitales Datensignal zu erzeugen, bei dem zumindest ein Teil der Nutzdaten zeitlich gestreckt ist, so dass ein zweiter Anteil einer zweiten Nutzdatendauer an der Auslesedauer größer ist als der erste Anteil.

Der Erfindung liegen u.a. die folgenden Überlegungen zugrunde. Bei dem Auslesen einer bildgebenden Fläche eines Bildsensors ergeben sich so genannte aktive Bereiche, in denen Nutzdaten ausgelesen werden, und so genannte Austastbereiche, in denen keine Datenausgabe erfolgt. Die Zeitdauer innerhalb derer ein Austastbereich durchlaufen wird, wird als Austastzeit bezeichnet. Dabei sind insbesondere die horizontale Austastzeit, die bei einem Wechsel von einer Bildzeile zur nächsten Bildzeile entsteht, und die vertikale Austastzeit zu nennen, die bei einem Wechsel von der letzten Bildzeile zurück zur ersten Bildzeile entsteht.

Betrachtet man einen vereinfachten dreizeiligen Bildsensor, so ergibt sich vereinfacht die Abfolge: Nutzdatendauer, horizontale Austastzeit, Nutzdatendauer, horizontale Austastzeit, Nutzdatendauer, vertikale Austastzeit, Nutzdatendauer etc. Gemäß dem Stand der Technik werden die Daten in gleicher Weise und mit der gleichen Frequenz übertragen, mit der das Auslesen der bildgebenden Fläche des Bildsensors erfolgt.

Im Rahmen der Erfindung wurde erkannt, dass gemäß dem Stand der Technik Übertragungsbandbreite auch dafür genutzt wird, die Austastzeiten zu übertragen, obwohl hier gar keine Bildinformation (Nutzdaten) vorhanden ist. Betrachtet man diese Situation entlang der Zeitachse, so ist festzustellen, dass ein erheblicher Anteil der zur Verfügung stehenden Übertragungszeit nur der Übertragung der Austastzeiten dient, was einerseits eine ineffektive Nutzung der Übertragungsbandbreite darstellt und andererseits eine unnötig hohe Übertragungsbandbreite erfordert.

Einer der Grundgedanken der vorliegenden Erfindung ist, dass man den Anteil der Nutzdaten bezogen auf eine bestimmte Übertragungsdauer erhöht bzw. den Anteil der Austastzeit bezogen auf eine bestimmte Gesamtdauer reduziert. Wie nachfolgend noch erläutert wird, kann auf diese Weise die erforderliche Übertragungsfrequenz über das Datenübertragungselement reduziert werden und können damit die Anforderungen an die Leistungsfähigkeit des Datenübertragungselements reduziert werden. Insbesondere ist es möglich, bei gegebenem Leitungsdurchmesser die Qualität und die Zuverlässigkeit der Bildübertragung zu verbessern oder aber unter Beibehaltung der Qualität und/oder Zuverlässigkeit der Bildübertragung den Querschnitt der Leitung bzw. des Datenübertragungselements zu reduzieren.

Als Beispiel soll ein Bildsensor dienen, bei dem die 1920 Pixel einer Zeile (Nutzdaten) innerhalb von 20 µs (erste Nutzdatendauer) ausgelesen werden, gefolgt von einer Austastzeit von 6,4 µs. Die gesamte Auslesedauer für eine Zeile beträgt demnach 20 µs + 6,4 µs = 26,4 µs. Der erste Anteil der ersten Nutzdatendauer bezogen auf die Auslesedauer beträgt für das erste digitale Datensignal demnach 20 µs / 26,4 µs = 76 %.

Werden die Nutzdaten nun so gestreckt, dass sich die Nutzdatendauer von der ersten Nutzdatendauer mit 20 µs auf eine zweite Nutzdatendauer von 26,4 µs erhöht - mit anderen Worten, es wird auch die gesamte Austastzeit zum Übertragen von Nutzdaten genutzt -, so ergibt sich nun ein zweiter Anteil der zweiten Nutzdatendauer an der Auslesedauer für das zweite digitale Datensignal von 26 µs / 26 µs = 100 %.

Zwar erlaubt es die Erfindung auch weiterhin, zusätzlich zu dem beschriebenen Konzept Datenkompression, Datenreduktion und/oder Multiplexing zu verwenden, doch wird ein besonderer Vorteil der Erfindung u.a. darin gesehen, dass ohne eine inhaltliche Veränderung der Nutzdaten eine Übertragung der Nutzdaten über das Datenübertragungselement technisch erleichtert wird.

Betrachtet man nun ein System gemäß dem Stand der Technik, welches die Nutzdaten mit der Pixelauslesefrequenz überträgt, so bietet die vorliegende Erfindung insbesondere die folgenden Vorteile. Soll der Durchmesser des Datenübertragungselements unverändert bleiben, so ermöglicht die Erfindung eine bessere Qualität und/oder Zuverlässigkeit bei der Übertragung der Nutzdaten. Andererseits ist es auch möglich, die Menge der Nutzdaten zu erhöhen, bspw. wenn man einen Bildsensor mit höherer Auflösung einsetzen möchte. Wenn weder die Qualität und/oder die Zuverlässigkeit der Übertragung der Nutzdaten noch die Menge der Nutzdaten erhöht werden soll, so eröffnet die Erfindung die Möglichkeit, den Querschnitt des Datenübertragungselements zu reduzieren. Zwar führt die Reduzierung des Durchmessers prinzipbedingt zu einer Erhöhung der Dämpfung, doch kann dieser nachteilige Effekt durch die zeitliche Streckung der Nutzdaten kompensiert oder sogar überkompensiert werden.

Vereinfacht gesagt macht sich die Erfindung die Tatsache zu Nutze, dass Bildsensoren konstruktionsbedingt eine gewisse Mindestzeit innerhalb einer Bildzeile benötigen, in der kein Auslesen aktiver Pixel erfolgt und somit auch keine Nutzdaten an das Datenübertragungselement geliefert werden. Die Erfindung nutzt nun diese inaktive Zeit ebenfalls für die Übertragung von Nutzdaten über das Datenübertragungselement.

Um die Erfindung einfacher erläutern zu können, wurde der Begriff der Auslesedauer eingeführt. Diese soll gewissermaßen als zeitliche Referenz dienen, um aufzuzeigen, dass die Erfindung eine effektivere Nutzung der zur Verfügung stehenden Zeit ermöglicht. Dabei kann die Auslesedauer prinzipiell auf jeden Teil des Auslesevorgangs bezogen werden, bei dem sowohl Nutzdaten gelesen als auch zumindest ein Teil einer Austastzeit erfasst ist. Bevorzugt wird die Auslesedauer als die Zeitspanne für das Auslesen von mindestens einer Zeile der bildgebenden Fläche verstanden. Dies eröffnet auch die Möglichkeit, die Auslesedauer auf das Auslesen von zwei, drei und mehr Zeilen zu beziehen, bis hin zum Auslesen der gesamten bildgebenden Fläche einschließlich der vertikalen Austastzeit.

Es sei darauf hingewiesen, dass sich die Erfindung auch realisieren lässt, wenn nur ein Teil der Nutzdaten zeitlich gestreckt ist. Dabei werden dann die Nutzdaten zeitlich gestreckt, die eine höhere Wichtigkeit gegenüber anderen Nutzdaten haben. Es wird aber derzeit als vorteilhaft angesehen, alle Nutzdaten zeitlich zu strecken, insbesondere im gleichen Maße zeitlich zu strecken, damit die Übertragung der Nutzdaten in das Datenübertragungselement hinein bei einer gleichbleibenden Frequenz erfolgt. Schließlich sei klargestellt, dass es sich bei der zeitlichen Streckung der Nutzdaten um eine zeitliche Streckung bei der zeitlichen Abfolge der Nutzdaten handelt. Das heißt, bezogen auf eine bestimmte absolute Zeitdauer werden im ersten Datensignal mehr Nutzdaten übertragen als - während derselben Zeitdauer - gestreckte Nutzdaten im zweiten Datensignal.

Schließlich sei darauf hingewiesen, dass im Rahmen der vorherigen Betrachtungen ein etwaiger Overhead, insbesondere ein Zeilen-Startcode, bei der Bestimmung von erstem Anteil und zweitem Anteil nicht berücksichtigt wurde, da der Anteil des Overheads bei der Übertragung einer Bildzeile bzw. bei der Übertragung eines Bildes gering ist. Ferner sollen unter den Nutzdaten im Rahmen der vorliegenden Erfindung insbesondere Bilddaten verstanden werden, die auf einem Bildschirm dargestellt werden sollen und für den Betrachter visuelle Informationen liefern.

Damit ist die Aufgabe vollständig gelöst.

Bei einer bevorzugten Ausgestaltung der Erfindung ist die Verarbeitungseinrichtung dafür ausgebildet, die zeitlich gestreckten Nutzdaten mit einer zweiten Frequenz zu übertragen, die geringer ist als eine erste Frequenz, bei der die Nutzdaten von der bildgebenden Fläche gelesen werden.

Bei dieser Ausgestaltung kann auf besonders einfache Weise die erforderliche Übertragungsfrequenz über das Datenübertragungselement reduziert werden und können damit die Anforderungen an die Leistungsfähigkeit des Datenübertragungselements reduziert werden, da die zeitliche Streckung der Nutzdaten einfach unter Verwendung einer zweiten Frequenz erfolgt, die geringer als die erste Frequenz ist.

Mit anderen Worten, es wird die Übertragungsfrequenz im Datenübertragungselement gesenkt. Durch die geringere Übertragungsfrequenz wird das Signal bei gleichem Leitungsdurchmesser weniger stark gedämpft, was eine Übertragung überhaupt erst ermöglicht, eine Übertragung über eine größere Länge zulässt und/oder eine Übertragung bei einem geringeren Leitungsdurchmesser zulässt.

Als Beispiel soll wieder der Bildsensor dienen, bei dem die 1920 Pixel einer Zeile (Nutzdaten) innerhalb von 20 µs ausgelesen werden, gefolgt von einer Austastzeit von 6,4 µs. Es sei ferner angenommen, dass der Bildsensor mit einer Auslesefrequenz von 96 MHz und einer Übertragungsrate von 960 Mbit/s arbeitet.

Da die Bilddaten der 1920 Pixel nun gemäß der Erfindung innerhalb von 26,4 µs übertragen werden können anstatt innerhalb von 20 µs, können die Bilddaten nun mit einer zweiten, geringeren Pixelfrequenz von 73 MHz und einer zweiten, geringeren Übertragungsrate von 730 Mbit/s in das Datenübertragungselement gesendet werden.

Bei einer weiteren bevorzugten Ausgestaltung der Erfindung weist die Verarbeitungseinrichtung eine Speichereinrichtung auf, die dazu ausgebildet ist, Daten mit einer ersten Datenrate aufzunehmen und mit einer zweiten Datenrate abzugeben, wobei die zweite Datenrate kleiner als die erste Datenrate ist.

Auf diese Weise lässt sich die zeitliche Streckung der Nutzdaten besonders einfach realisieren. Allgemein gesprochen wird das erste Datensignal mit der Auslesefrequenz beim Auslesen der bildgebenden Fläche in die Speichereinrichtung geschrieben und das zweite Datensignal langsamer, also mit den zeitlich gestreckten Nutzdaten, aus der Speichereinrichtung ausgelesen.

Mittels der Speichereinrichtung können zudem auf einfache Weise verschiedene Techniken angewandt werden, um die Austastzeiten zu reduzieren bzw. zu unterdrücken. Werden während des Auslesens der bildgebenden Fläche noch Daten geliefert, bei denen es sich aber nicht um Nutzdaten handelt, so ist es bevorzugt, wenn diese Austastdaten nicht in die Speichereinrichtung geschrieben werden, das heißt, die Speichereinrichtung akzeptiert die Austastdaten nicht. Dieses Ausblenden kann durch eine Analyse der Datenwerte, insbesondere mittels eines Schwellwerts, oder in Kenntnis des Beginns und des Endes der Austastzeit durchgeführt werden.

Werden die Austastdaten doch zunächst in die Speichereinrichtung geschrieben, so können diese in ähnlicher Weise beim Auslesen der Speichereinrichtung für das zweite Datensignal ausgeblendet werden. Besonders bevorzugt ist es, wenn während der Austastzeit gar keine Daten an die Speichereinrichtung gesendet werden, da dann kein Ausblenden der Austastdaten in der Speichereinrichtung benötigt wird.

Bei einer weiteren vorteilhaften Ausgestaltung der Erfindung weist die Speichereinrichtung ein FIFO auf oder ist von einem FIFO gebildet.

Diese Ausgestaltung ist vorteilhaft, weil die Speichereinrichtung mit einem FIFO (First-In-First-Out) besonders kostengünstig realisiert werden kann. Das FIFO hat dabei eine Größe, um die vollständigen Informationen von mindestens 10, bevorzugt mindestens 50, besonders bevorzugt 100 und insbesondere mindestens 250 Pixeln zu speichern.

Mit diesen Größenangaben soll deutlich gemacht werden, dass es sich bei dem erfindungsgemäßen FIFO nicht um ein kleines FIFO handelt, das der Formatierung von Daten dient, wie z.B. der Aufteilung von 10-Bit-Pixeldaten in 8-Bit-Bytepakete und wie es regelmäßig in Bildsensoren vorhanden ist. Vielmehr ermöglicht erst ein FIFO mit der genannten Mindestgröße eine effektive Nutzung der erfindungsgemäßen Lehre.

Dabei ist es vorteilhaft, wenn die Größe des FIFO in Pixeln so groß ist, dass alle Pixeldaten, also alle Nutzdaten, die im zweiten Datensignal während der Austastzeit übertragen werden, gespeichert werden können. Soll die gesamte Austastzeit für die Übertragung von Nutzdaten im zweiten Datensignal genutzt werden-wieder unter Vernachlässigung des oben genannten Overheads -, so erhält man die Mindestgröße des FIFO in Pixeln, indem man die horizontale Austastzeit mit der zweiten Datenrate des zweiten Datensignals multipliziert. Wenn die horizontale Austastzeit 6,4 µs und die zweite Datenrate 73 Mbyte/s beträgt, muss die Größe des FIFO mindestens 468 Pixel betragen, wenn man 10 Bit je Pixel annimmt. Soll außerdem noch die vertikale Austastzeit genutzt werden, so muss die Größe des FIFO ggf. vergrößert werden.

Bei einer weiteren vorteilhaften Ausgestaltung entspricht ein erster Quotient von zweitem Anteil und erstem Anteil zumindest in etwa einem zweiten Quotienten von Auslesedauer und erster Nutzungsdauer.

Bei dieser Ausgestaltung wird das zweite Datensignal derart erzeugt, dass zumindest in etwa die gesamte Auslesedauer, also einschließlich der Austastzeit, zum Übertragen von Nutzdaten verwendet wird. Auf diese Weise kann die zweite Datenrate des zweiten Datensignals bzw. die zweite Frequenz des zweiten Datensignals besonders stark reduziert werden.

Bei einer weiteren bevorzugten Ausgestaltung der Erfindung ist der erste Anteil kleiner als 95 %, bevorzugt kleiner als 90 %, besonders bevorzugt kleiner als 85 % und insbesondere kleiner als 75 % und/oder ist der zweite Anteil größer als 85 %, bevorzugt größer als 90 %, besonders bevorzugt größer als 95 % und insbesondere größer als 98 %.

Wenngleich sich die Erfindung auch bei besonders leistungsfähigen Bildsensoren, die bereits eine sehr geringe Austastzeit haben, vorteilhaft einsetzen lässt, so zeigt sich die Leistungsfähigkeit der vorliegenden Erfindung insbesondere bei solchen Bildsensoren, die eine relativ große Austastzeit haben, mit anderen Worten, bei denen der erste Anteil deutlich geringer als 100 % ist. Ferner, auch wenn es für die Nutzung der Erfindung nicht erforderlich ist, die Nutzdaten auf die gesamte Auslesedauer zu strecken, so zeigt sich die Leistungsfähigkeit der Erfindung insbesondere dann, wenn der zweite Anteil in Richtung von 100 % gewählt wird.

Bei den zuvor genannten Zahlenwerten wurde ein etwaiger Overhead, insbesondere ein Zeilen-Startcode, bei der Bestimmung von erstem Anteil und zweitem Anteil nicht berücksichtigt. Möchte man dies dennoch tun, so fallen die Prozentwerte um den Anteil geringer aus, den der Overhead bezogen auf die Auslesedauer hat. Eine vollständige (100%) Nutzung der Auslesedauer für Nutzdaten ist dann nicht mehr möglich.

Bei einer weiteren vorteilhaften Ausgestaltung der Erfindung entspricht das Verhältnis von zweiter Datenrate zu erster Datenrate zumindest in etwa dem Verhältnis von erster Nutzdatendauer zu Auslesedauer.

Auch diese Ausgestaltung stellt darauf ab, dass zumindest in etwa die gesamte Auslesedauer zum Übertragen von Nutzdaten verwendet wird, mit anderen Worten, dass zumindest in etwa auch die Austastzeit zum Übertragen von Nutzdaten verwendet wird.

Bei einer weiteren vorteilhaften Ausgestaltung der Erfindung weist die Ausleseeinrichtung einen Ausleseausgang auf und weist die Verarbeitungseinrichtung einen Verarbeitungseingang auf, wobei der Ausleseausgang und der Verarbeitungseingang miteinander verbunden sind.

Bevorzugt sind der Ausleseausgang und der Verarbeitungseingang direkt miteinander verbunden, um eine schnelle und störungsfreie Datenübertragung zu gewährleisten. Eventuelle Bauelemente, die für den Betrieb der bildgebenden Vorrichtung erforderlich sind, wie bspw. ein A/D-Wandler zum Wandeln der analogen Nutzdaten, die von der bildgebenden Fläche gelesen werden, in das erste digitale Datensignal, werden der Ausleseeinrichtung oder der Verarbeitungseinrichtung zugeordnet.

Bei einer weiteren vorteilhaften Ausgestaltung der Erfindung ist die Verarbeitungseinrichtung derart ausgebildet, dass Nutzdaten zeitlich so gestreckt werden, dass die Nutzdatendauer zumindest in etwa einer Zeitspanne für das Auslesen von mindestens einer Zeile der bildgebenden Fläche einschließlich der zugehörigen Austastzeit entspricht.

Auch diese Ausgestaltung zielt darauf ab, möglichst viel der - ansonsten ungenutzten - Austastzeit für die Übertragung von Nutzdaten zu verwenden.

Bei einer anderen Anordnung weist die Vorrichtung zusätzlich zu dem genannten, ersten, Bildsensor einen zweiten Bildsensor, zusätzlich zu der genannten, ersten, Verarbeitungseinrichtung eine zweite Verarbeitungseinrichtung, einen Bildsignalausgang und eine Synchronisationseinrichtung auf, wobei die Synchronisationseinrichtung abwechselnd die erste und die zweite Verarbeitungseinrichtung mit dem Bildsignalausgang verbindet.

Diese Ausgestaltung zeigt, dass auch eine Verwendung von zwei Bildsensoren möglich ist. Das FIFO wird hier allerdings nicht dazu verwendet, um ein zweites Datensignal mit einem zweiten Anteil von zweiter Nutzdatendauer an der Auslesedauer zu erzeugen, sondern um Daten zwischenzuspeichern, wenn die Synchronisationseinrichtung gerade die jeweils andere Verarbeitungseinrichtung mit dem Bildsignalausgang verbunden hat.

Gemäß einem zweiten Aspekt der Erfindung wird die Aufgabe gelöst durch ein endoskopisches Instrument mit einem Schaft, der ein distales Ende, an dem mindestens eine zuvor beschriebene, bildgebende Vorrichtung angeordnet ist, und ein proximales Ende zum Anschluss an eine Versorgungseinheit aufweist, und mit einem Datenübertragungselement, das zwischen der mindestens einen bildgebenden Vorrichtung und dem proximalen Ende angeordnet ist.

Ein solches endoskopisches Instrument lässt sich besonders kompakt bezüglich des Durchmessers bauen, da die zeitlich gestreckten Nutzdaten mit einer verringerten zweiten Frequenz über das Datenübertragungselement übertragen werden.

Gemäß einem dritten Aspekt der Erfindung wird die Aufgabe gelöst durch ein Verfahren zum Betreiben einer bildgebenden Vorrichtung, insbesondere einer zuvor beschriebenen, bildgebenden Vorrichtung, wobei das Verfahren die folgenden Schritte aufweist:
- Auslesen von mindestens einer Zeile eines Bildsensors über eine Auslesedauer,
- Erzeugen eines ersten digitalen Datensignals mit Nutzdaten, der einen ersten Anteil einer ersten Nutzdatendauer bezogen auf die Auslesedauer aufweist,
- Erzeugen eines zweiten digitalen Datensignals auf der Basis des ersten Datensignals, wobei bei dem zweiten Datensignal zumindest ein Teil der Nutzdaten zeitlich gestreckt ist, so dass ein zweiter Anteil einer zweiten Nutzdatendauer an der Auslesedauer größer ist als der erste Anteil.

Bei einer bevorzugten Ausgestaltung der Erfindung trägt das zweite Datensignal auch zu solchen Zeiten Nutzdaten, zu denen der Schritt des Auslesens keine Nutzdaten liefert.

Bei einer weiteren vorteilhaften Ausgestaltung der Erfindung ist eine zweite Frequenz des zweiten Datensignals kleiner als eine erste Frequenz des ersten Datensignals.

Gemäß einem vierten Aspekt der vorliegenden Erfindung wird die Aufgabe gelöst durch eine Verwendung einer zuvor beschriebenen, bildgebenden Vorrichtung für eine Verarbeitung eines ersten Datensignals mit Nutzdaten, die mit einer ersten Frequenz von einer bildgebenden Fläche des Bildsensors ausgelesen werden, in ein zweites Datensignal mit Nutzdaten, der eine zweite Frequenz hat, die geringer als die erste Frequenz ist.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung näher dargestellt und werden in der nachfolgenden Beschreibung näher erläutert. Es zeigen:
- Fig. 1: ein endoskopisches Instrument;
- Fig. 2: eine erste Ausführungsform einer bildgebenden Vorrichtung;
- Fig. 3: ein Verfahren zum Betreiben einer bildgebenden Vorrichtung;
- Fig. 4: ein Timingdiagramm bezüglich der Funktionsweise der bildgebenden Vorrichtung gemäß Fig. 2;
- Fig. 5: ein Timingdiagramm bezüglich einer ersten alternativen Funktionsweise der bildgebenden Vorrichtung gemäß Fig. 2;
- Fig. 6: ein Timingdiagramm bezüglich einer zweiten alternativen Funktionsweise der bildgebenden Vorrichtung gemäß Fig. 2;
- Fig. 7: ein Timingdiagramm bezüglich einer dritten alternativen Funktionsweise der bildgebenden Vorrichtung gemäß Fig. 2;
- Fig. 8: eine zweite Ausführungsform der bildgebenden Vorrichtung;
- Fig. 9: eine andere bildgebende Vorrichtung; und
- Fig. 10: ein Timingdiagramm bezüglich der Funktionsweise der bildgebenden Vorrichtung gemäß Fig. 9.

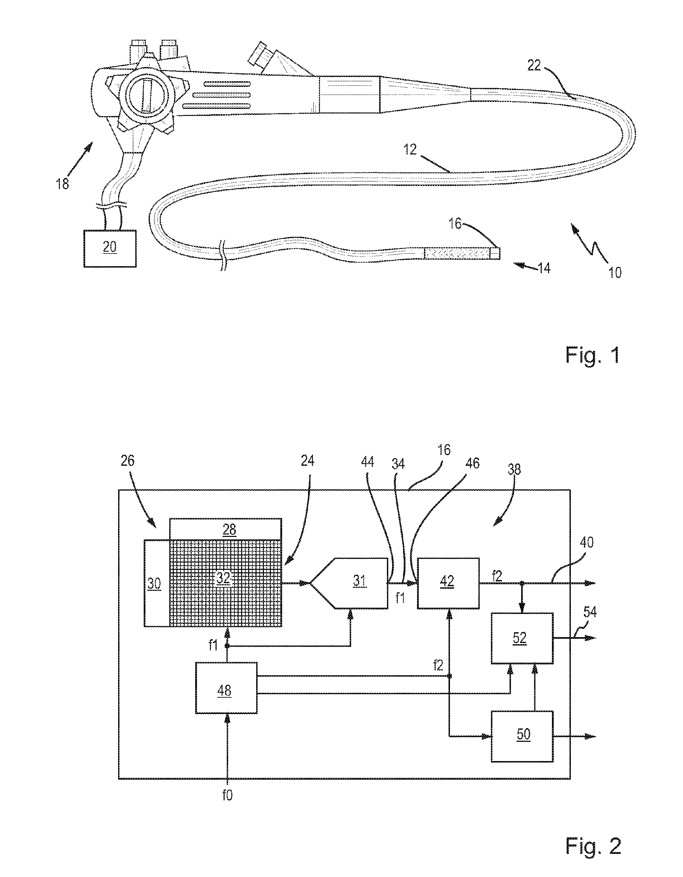

Fig. 1 zeigt eine Ausführungsform eines endoskopischen Instruments 10. Das endoskopische Instrument 10, das in der dargestellten Ausführungsform als Endoskop ausgebildet ist, weist einen Schaft 12 auf, der ein distales Ende 14, an dem eine bildgebende Vorrichtung 16 angeordnet ist, und ein proximales Ende 18 zum Anschluss an eine Versorgungseinheit 20 aufweist. Zwischen dem optischen Sensor 16 und dem proximalen Ende 18 ist ein Datenübertragungselement 22 angeordnet.

Das Datenübertragungselement 22 ist dafür ausgebildet, mindestens Signale von der bildgebenden Vorrichtung 16 zu übertragen, insbesondere differentiell zu übertragen. Das endoskopische Instrument 10 ist an seinem proximalen Ende 18 mit der Versorgungseinheit 20 verbunden. Außerdem kann dem optischen Sensor 16 mindestens eine optische Einheit (nicht dargestellt) zugeordnet sein, insbesondere eine Anordnung von Stablinsen, die distalseitig bzw. strahlseitig vor der mindestens einen bildgebenden Vorrichtung 16 angeordnet ist. Die bildgebende Vorrichtung 16 muss also nicht zwingend den distalseitigen Abschluss des endoskopischen Instruments 10 bilden.

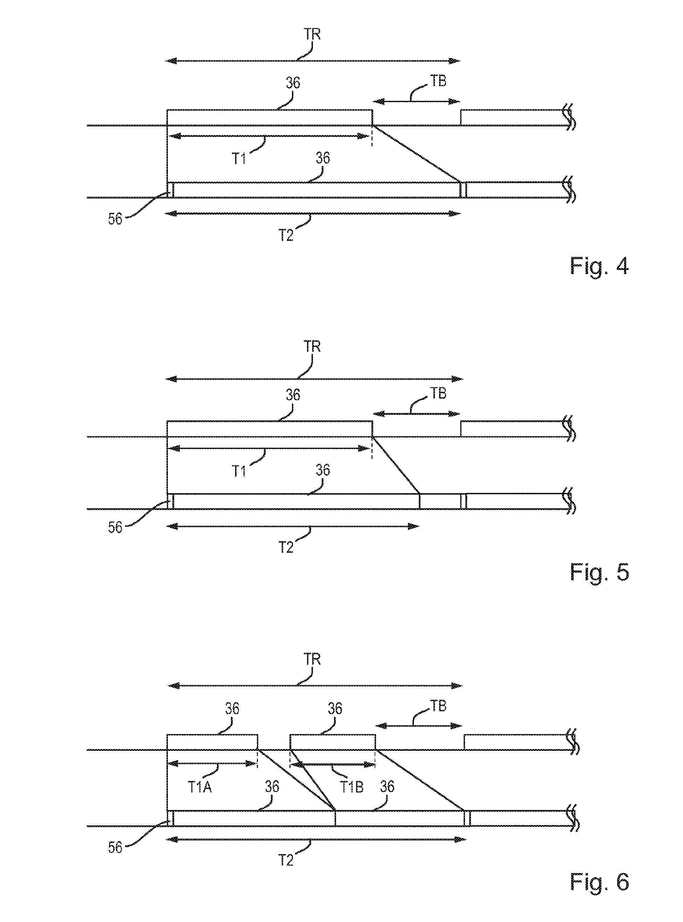

Fig. 2 zeigt eine Ausführungsform einer bildgebenden Vorrichtung 16 für ein endoskopisches Instrument 10, insbesondere gemäß Fig. 1. Die Vorrichtung 16 hat einen Bildsensor 24, der eine Ausleseeinrichtung 26, hier mit einer Spaltenauswahleinrichtung 28 und einer Zeilenauswahleinrichtung 30, zum Auslesen einer bildgebenden Fläche 32 des Bildsensor 24 aufweist. Die Ausleseeinrichtung 26 ist dafür ausgebildet, mittels eines Analog/Digital-Konverters 31 ein erstes digitales Datensignal 34 mit Nutzdaten 36 (siehe Fig. 4) bei einer ersten Frequenz f1 zu erzeugen, der einen ersten Anteil P1 einer ersten Nutzdatendauer T1 (siehe Fig. 4) bezogen auf eine Auslesedauer TR (siehe Fig. 4)aufweist.

Die bildgebende Vorrichtung 16 weist außerdem eine Verarbeitungseinrichtung 38 auf, die dafür ausgebildet ist, auf der Basis des ersten Datensignals 34 ein zweites digitales Datensignal bei einer zweiten Frequenz f2 zu erzeugen, bei dem zumindest ein Teil der Nutzdaten 36 zeitlich gestreckt ist, so dass ein zweiter Anteil P2 einer zweiten Nutzdatendauer T2 (siehe Fig. 4) an der Auslesedauer TR größer ist als der erste Anteil P1.

Die zeitlich gestreckten Nutzdaten 36 werden mit einer zweiten Frequenz f2 übertragen, die geringer ist als eine erste Frequenz f1, bei der die Nutzdaten 36 von der bildgebenden Fläche 32 gelesen werden. Die Bearbeitungseinrichtung 38 weist eine Speichereinrichtung 42 auf, die dafür ausgebildet ist, Daten mit einer ersten Datenrate aufzunehmen und mit einer zweiten Datenrate abzugeben, wobei die zweite Datenrate kleiner als die erste Datenrate ist. Die Speichereinrichtung 42 ist hier als FIFO ausgebildet. Bei der hier gezeigten Ausführungsform entspricht das Verhältnis von zweiter Datenrate zu erster Datenrate zumindest in etwa dem Verhältnis von erster Nutzdatendauer T1 zu Auslesedauer TR.

Die Ausleseeinrichtung 26 weist einen Ausleseausgang 44 auf, und die Verarbeitungseinrichtung 38 weist einen Verarbeitungseingang 46 auf, wobei der Ausleseausgang 44 und der Verarbeitungseingang 46 miteinander verbunden sind, hier direkt miteinander verbunden sind. Die Verarbeitungseinrichtung 38 ist hier derart ausgebildet, dass Nutzdaten 36 zeitlich so gestreckt werden, dass die zweite Nutzdatendauer T2 zumindest in etwa einer Zeitspanne für das Auslesen von mindestens einer Zeile der bildgebenden Fläche 32 einschließlich der zugehörigen Austastzeit TB (siehe Fig. 4) entspricht.

Die weiteren Details der bildgebenden Vorrichtung 16 können im Hinblick auf die jeweiligen Zielvorgaben variabel gestaltet werden. Bei der hier gezeigten Ausführungsform wurde die folgende Realisierung gewählt:

Der Bildsensor 16 wird von einem Systemtakt f0 gespeist, der an eine PLL-Einheit 48 (Phase Locked Loop) geleitet wird. Die PLL-Einheit 48 erzeugt eine erste Frequenz f1, die zum Bildsensor 24 geführt wird, und eine zweite Frequenz f2, die zur Speichereinrichtung 42 und zu einer Timingsteuerung 50 geführt wird, die für die horizontale und vertikale Synchronisation zuständig ist. Ferner befindet sich auf der bildgebenden Vorrichtung 16 ein Serialisierer 52, der neben dem parallelen zweiten Datensignal 40 ein weiteres serielles Datensignal 54 erzeugt. Es sei darauf hingewiesen, dass in Abhängigkeit von der Implementierung auch das erste Datensignal 40 unmittelbar als serielles Datensignal ausgestaltet werden kann.

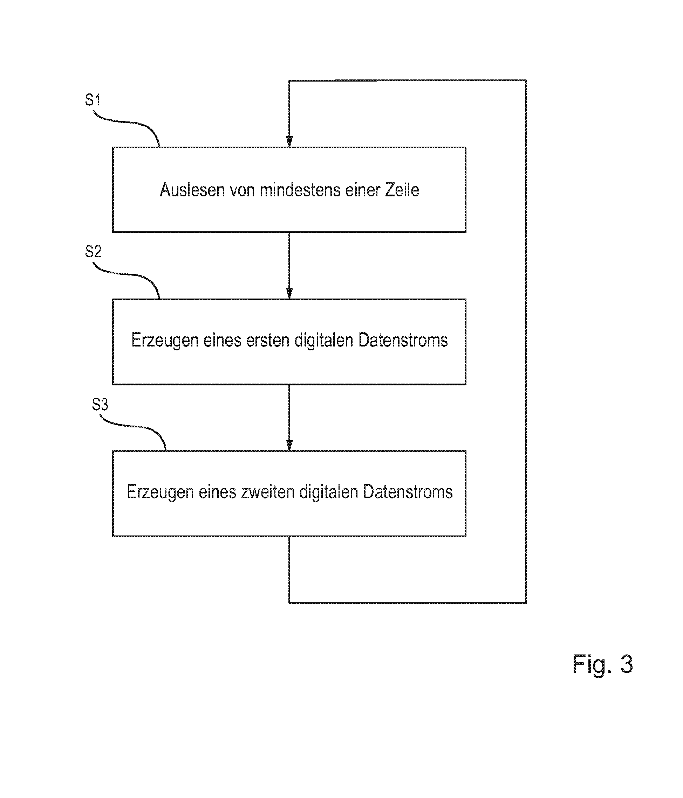

Die Funktionsweise der bildgebenden Vorrichtung 16 soll nun anhand der Fig. 3 erläutert werden. In einem ersten Schritt S1 wird mindestens eine Zeile des Bildsensors 24 über die Auslesedauer TR ausgelesen. Im Schritt S2 wird ein digitales Datensignal 34 mit Nutzdaten 36 erzeugt, der einen ersten Anteil P1 einer ersten Nutzdatendauer T1 bezogen auf die Auslesedauer TR aufweist. In einem dritten Schritt S3 wird dann ein zweites digitales Datensignal 40 auf der Basis des ersten Datensignals 34 erzeugt, wobei bei dem zweiten Datensignal 40 zumindest ein Teil der Nutzdaten 36 zeitlich gestreckt ist, so dass ein zweiter Anteil P2 einer zweiten Nutzdatendauer T2 an der Auslesedauer TR größer ist als der erste Anteil P1.

Fig. 4 zeigt ein Timingdiagramm bezüglich der Funktionsweise der bildgebenden Vorrichtung gemäß Fig. 2. Der obere Teil der Darstellung zeigt das erste digitale Datensignal 34 während der Auslesedauer TR einschließlich der Austastzeit TB. Die untere Darstellung zeigt das zweite Datensignal 40, wobei hier der Vollständigkeit halber auch ein Zeilen-Startcode 56 dargestellt ist, dem jedoch für eine vereinfachte Erläuterung keine zeitliche Relevanz zugemessen wird und der nicht zu den Nutzdaten 36 gerechnet wird. Zwischen der oberen und unteren Darstellung sind Verbindungslinien eingezeichnet, um die zeitliche Streckung der Nutzdaten 36 zu visualisieren.

Es sei erneut darauf hingewiesen, dass die Nutzdaten 36 im ersten Datensignal bestimmten Veränderungen unterworfen werden können, z.B. Datenreduktion oder Datenkompression, bevor sie in das zweite Datensignal 40 aufgenommen werden. Die Erfindung bietet aber gerade die Möglichkeit, auf derartige Manipulationen innerhalb der bildgebenden Vorrichtung 16 verzichten zu können und die Nutzdaten 36 unverändert - die zeitliche Steckung verändert die Nutzdaten nicht - in das zweite Datensignal 40 überführen zu können.

Anhand des bereits vorher erwähnten Beispiels, T1 = 20 µs, TR = T2 = 26,4 µs und TB = 6,4 µs, ergeben sich ein erster Anteil P1 = T1/TR = 76 % und P2 = T2/TR = 100 %. Bei der hier gezeigten Ausführungsform entspricht ein erster Quotient Q1 von zweitem Anteil P2 und erstem Anteil P1 zumindest in etwa einem zweiten Quotienten Q2 von Auslesedauer TR und erster Nutzungsdauer T1. Die technische Wirkung der Erfindung ist besonders vorteilhaft, wenn der erste Anteil P1 kleiner als 95%, bevorzugt kleiner 90%, besonders bevorzugt kleiner als 85 % und insbesondere kleiner als 75 % ist und/oder der zweite Anteil P2 größer als 85 %, bevorzugt größer als 90 %, besonders bevorzugt größer als 95 % und insbesondere größer als 98 % ist. Selbstverständlich liegt auch diesen Angaben immer zugrunde, dass der zweite Anteil größer als der erste Anteil ist.

Fig. 5 zeigt ein Timingdiagramm bezüglich einer ersten alternativen Funktionsweise der bildgebenden Vorrichtung gemäß Fig. 2. Es gelten auch hier die zuvor gemachten Erläuterungen, jedoch wurde der zweite Anteil P2 etwas geringer gewählt, ca. 90 %, mit anderen Worten, die zeitliche Streckung der Nutzdaten 36 im Vergleich zur Fig. 4 verringert.

Fig. 6 zeigt ein Timingdiagramm bezüglich einer zweiten alternativen Funktionsweise der bildgebenden Vorrichtung gemäß Fig. 2. Auch hier gelten die zuvor gemachten Erläuterungen, wobei bei dieser Darstellung angenommen wurde, dass die Nutzdaten 36 während einer Auslesedauer TR unterbrochen werden. Durch die zeitliche Streckung der Nutzdaten 36 werden die Unterbrechung und die Austastzeit vollständig genutzt. Die erste Nutzdatendauer T1 setzt sich hier aus den beiden Teilzeiten T1A und T1B zusammen. Bei dieser Ausgestaltung liegt der erste Anteil P1 bei ca. 65 % und der zweite Anteil P2 bei ca. 100 %. Es sei darauf hingewiesen, dass es grundsätzlich möglich ist, die zeitliche Streckung der Nutzdaten während der Zeit T1A anders zu wählen als die zeitliche Streckung der Nutzdaten 36 während der Dauer T1B.

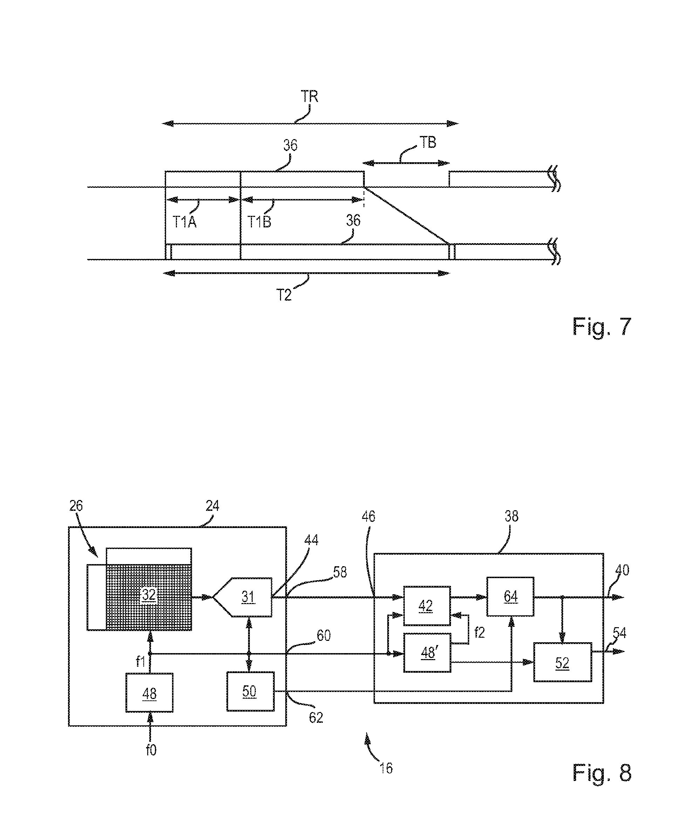

Fig. 7 zeigt ein Timingdiagramm bezüglich einer dritten alternativen Funktionsweise der bildgebenden Vorrichtung gemäß Fig. 2. Hier ist ein denkbarer Anwendungsfall dargestellt, bei dem lediglich ein Teil der Nutzdaten 36 zeitlich gestreckt werden soll. Auch hier setzt sich die erste Nutzdatendauer T1 aus den beiden Teildauern T1A und T1B zusammen, wobei hier nur eine zeitliche Streckung der Nutzdaten 36 während der Dauer T1B erfolgt.

Fig. 8 zeigt eine zweite Ausführungsform der bildgebenden Vorrichtung 16. Dabei gelten prinzipiell auch die vorherigen Erläuterungen. Die Elemente, die in ihrer Funktion gleich sind, wurden wieder mit den bereits eingeführten Bezugszeichen versehen.

Die zweite Ausführungsform unterscheidet sich von der ersten Ausführungsform gemäß Fig. 2 dadurch, dass die Verarbeitungseinrichtung 38 als PLD (Programmable Logic Device) realisiert wurde, und dass es sich bei dem Bildsensor 24 um einen handelsüblichen Bildsensor mit einem ersten Ausgang 58 für parallele Videodaten, einem zweiten Ausgang 60 für die Auslesefrequenz f1 und einen dritten Ausgang 62 für Synchronisationsinformation (horizontale und vertikale Synchronisation) handelt.

Die Verarbeitungseinrichtung 38 wurde um eine zweite PLL-Einheit 48' erweitert, die innerhalb der Verarbeitungseinrichtung 38 die zweite Frequenz f2 erzeugt. Außerdem ist auf der Verarbeitungseinrichtung 38 eine Synchronworteinheit 64 angeordnet, die die Synchronisationsinformation vom Bildsensor 24 in das zweite Datensignal 40 einfügt.

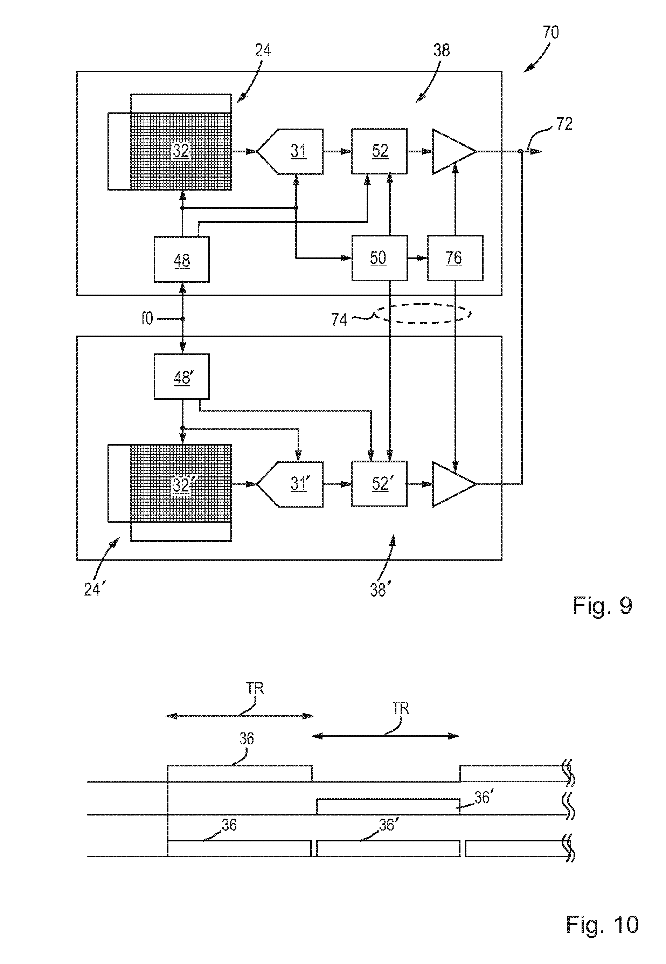

Fig. 9 zeigt eine bildgebende Vorrichtung 70, die zusätzlich zu dem bereits genannten, ersten, Bildsensor 24 einen zweiten Bildsensor 24', zusätzlich zu der genannten, ersten Verarbeitungseinrichtung 38 eine zweite Verarbeitungseinrichtung 38', einen Bildsignalausgang 72 und eine Synchronisationseinrichtung 74 aufweist, wobei die Synchronisationseinrichtung 74 abwechselnd die erste und die zweite Verarbeitungseinrichtung 38, 38' mit dem Bildsignalausgang 72 verbindet. Die Vorrichtung 70 kann aufgrund ihrer Funktionsweise auf eine Streckung der Nutzdaten 36 verzichten, doch kann die Vorrichtung 70 auch so erweitert werden, dass eine Streckung der Nutzdaten 36 stattfindet.

Die Funktionsweise der Vorrichtung 70 erschließt sich im Hinblick auf die bereits gemachten Erläuterungen aus der Figur. Es sei lediglich darauf hingewiesen, dass die Synchronisation, ob am Bildsignalausgang 72 das Bild des ersten Bildsensors 24 oder das Bild des zweiten Bildsensors 24' anliegt, über die Synchronisationsschnittstelle 74 und die Multiplexsteuerung 76 gesteuert wird.

Das Ergebnis dieser Steuerung ist dem Timingdiagramm gemäß Fig. 10 zu entnehmen. Das obere Diagramm zeigt den Verlauf der Nutzdaten 36 vom ersten Bildsensor 24, das mittlere Diagramm zeigt den Verlauf der zweiten Nutzdaten 36' vom zweiten Bildsensor 24', und das untere Diagramm zeigt das Ausgangssignal am Bildsignalausgang 72.

Damit wurde insgesamt eine bildgebende Vorrichtung 16 aufgezeigt, die es ermöglicht, ein Bildsignal mit geringeren Anforderungen an das Datenübertragungselement 22 zu übertragen. Außerdem wurde eine Vorrichtung 70 aufgezeigt, die auf einfache Weise die Nutzung eines gemeinsamen Datenübertragungselements für zwei Bildsensoren ermöglicht.

## Patentansprüche

1. Bildgebende Vorrichtung (16) für ein endoskopisches Instrument (10), wobei die Vorrichtung (16) einen Bildsensor (24) aufweist, der eine Ausleseeinrichtung (26) zum Auslesen einer bildgebenden Fläche (32) des Bildsensors (24) aufweist, wobei die Ausleseeinrichtung (26) dafür ausgebildet ist, ein erstes digitales Datensignal (34) mit Nutzdaten (36) zu erzeugen, der einen ersten Anteil (P1) einer ersten Nutzdatendauer (T1) bezogen auf eine Auslesedauer (TR) aufweist, und wobei die Vorrichtung (16) eine Verarbeitungseinrichtung (38) aufweist, die dafür ausgebildet ist, auf der Basis des ersten Datensignals (34) ein zweites digitales Datensignal (40) zu erzeugen, bei dem zumindest ein Teil der Nutzdaten (36) zeitlich gestreckt ist, so dass ein zweiter Anteil (P2) einer zweiten Nutzdatendauer (T2) an der Auslesedauer (TR) größer ist als der erste Anteil (P1).

2. Vorrichtung nach Anspruch 1, wobei die Verarbeitungseinrichtung (38) dafür ausgebildet ist, die zeitlich gestreckten Nutzdaten (36) mit einer zweiten Frequenz (f2) zu übertragen, die geringer ist als eine erste Frequenz (f1), bei der die Nutzdaten (36) von der bildgebenden Fläche (32) gelesen werden.

3. Vorrichtung nach einem der vorhergehenden Ansprüche, wobei die Verarbeitungseinrichtung eine Speichereinrichtung (42) aufweist, die dafür ausgebildet ist, Daten mit einer ersten Datenrate aufzunehmen und mit einer zweiter Datenrate abzugeben, wobei die zweite Datenrate kleiner als die erste Datenrate ist.

4. Vorrichtung nach einem der vorhergehenden Ansprüche, wobei die Speichereinrichtung (42) ein FIFO aufweist oder von einem FIFO gebildet ist.

5. Vorrichtung nach einem der vorhergehenden Ansprüche, wobei ein erster Quotient (Q1) von zweitem Anteil (P2) und erstem Anteil (P1) zumindest in etwa einem zweiten Quotienten (Q2) von Auslesedauer (TR) und erster Nutzungsdatendauer (T1) entspricht.

6. Vorrichtung nach einem der vorhergehenden Ansprüche, wobei der erste Anteil (P1) kleiner als 95%, bevorzugt kleiner als 90%, besonders bevorzugt kleiner als 85% und insbesondere kleiner als 75% ist und/oder der zweite Anteil (P2) größer als 85%, bevorzugt größer als 90%, besonders bevorzugt größer als 95% und insbesondere größer als 98% ist.

7. Vorrichtung nach einem der vorhergehenden Ansprüche, wobei das Verhältnis von zweiter Datenrate zu erster Datenrate zumindest in etwa dem Verhältnis von erster Nutzdatendauer (T1) zu Auslesedauer (TR) entspricht.

8. Vorrichtung nach einem der vorhergehenden Ansprüche, wobei die Ausleseeinrichtung (26) einen Ausleseausgang (42) aufweist und die Verarbeitungseinrichtung (38) einen Verarbeitungseingang (44) aufweist, wobei der Ausleseausgang (42) und der Verarbeitungseingang (44) miteinander verbunden sind.

9. Vorrichtung nach einem der vorhergehenden Ansprüche, wobei die Verarbeitungseinrichtung (38) derart ausgebildet ist, dass Nutzdaten (36) zeitlich so gestreckt werden, dass die zweite Nutzdatendauer (T2) zumindest in etwa einer Zeitspanne für das Auslesen von mindestens einer Zeile der bildgebenden Fläche (32) einschließlich der zugehörigen Austastzeit (TB) entspricht.

10. Vorrichtung nach einem der vorhergehenden Ansprüche, wobei die Vorrichtung (70) zusätzlich zu dem genannten, ersten, Bildsensor (24) einen zweiten Bildsensor (24'), zusätzlich zu der genannten, ersten, Verarbeitungseinrichtung (38) eine zweite Verarbeitungseinrichtung (38'), einen Bildsignalausgang (72) und eine Synchronisationseinrichtung (74) aufweist, wobei die Synchronisationseinrichtung (74) abwechselnd die erste und die zweite Verarbeitungseinrichtung (38, 38') mit dem Bildsignalausgang (72) verbindet.

11. Endoskopisches Instrument (10) mit einem Schaft (12), der ein distales Ende (14), an dem mindestens eine bildgebende Vorrichtung (16) nach einem der Ansprüche 1 bis 10 angeordnet ist, und ein proximales Ende (18) zum Anschluss an eine Versorgungseinheit (20) aufweist, und mit einem Datenübertragungselement (22), das zwischen der mindestens einen bildgebenden Vorrichtung (16) und dem proximalen Ende (18) angeordnet ist.

12. Verfahren zum Betreiben einer bildgebenden Vorrichtung, insbesondere einer Vorrichtung (16) nach einem der Ansprüche 1 bis 10, wobei das Verfahren die folgenden Schritte aufweist:
- Auslesen (S1) von mindestens einer Zeile eines Bildsensors (24) über eine Auslesedauer (TR),
- Erzeugen (S2) eines ersten digitalen Datensignals (34) mit Nutzdaten (36), der einen ersten Anteil (P1) einer ersten Nutzdatendauer (T1) bezogen auf die Auslesedauer (TR) aufweist,
- Erzeugen (S3) eines zweiten digitalen Datensignals (40) auf der Basis des ersten Datensignals (34), wobei bei dem zweiten Datensignal (40) zumindest ein Teil der Nutzdaten (36) zeitlich gestreckt ist, so dass ein zweiter Anteil (P2) einer zweiten Nutzdatendauer (T2) an der Auslesedauer (TR) größer ist als der erste Anteil (P1).

13. Verfahren nach Anspruch 12, wobei das zweite Datensignal (40) auch zu solchen Zeiten Nutzdaten (36) trägt, zu denen der Schritt des Auslesens (S1) keine Nutzdaten (36) liefert.

14. Verfahren nach Anspruch 12 oder 13, wobei eine zweite Frequenz (f2) des zweiten Datensignals (40) kleiner ist als eine erste Frequenz (f1) des ersten Datensignals (34).

15. Verwendung einer bildgebenden Vorrichtung (16) nach einem der Ansprüche 1 bis 10 für eine Verarbeitung eines ersten Datensignals (34) mit Nutzdaten (36), die mit einer ersten Frequenz (f1) von einer bildgebenden Fläche (32) des Bildsensors (24) ausgelesen werden, in ein zweites Datensignal (40) mit Nutzdaten (36), der eine zweite Frequenz (f2) hat, die geringer als die erste Frequenz (f1) ist.
